# EUROPEAN PATENT SPECIFICATION

(11) **EP 1 299 587 B1**
(45) Date of publication and mention of the grant of the patent: **01.10.2008**
(21) Application number: 01984171.7
(22) Date of filing: 11.07.2001
(51) Int. Cl.: D04H 1/00, D04H 1/46, D04H 3/10, B32B 3/10

(54) **MULTI-COMPONENT NONWOVEN FABRIC FOR USE IN DISPOSABLE ABSORBENT ARTICLES**
MEHRKOMPONENTIGER VLIESSTOFF FÜR SAUGFÄHIGE WEGWERFARTIKEL
NON-TISSE A PLUSIEURS COMPOSANTS S'UTILISANT DANS DES ARTICLES ABSORBANTS JETABLE

(30) Priority: 11.07.2000 US 217523 P
(43) Date of publication of application: 09.04.2003
(73) Proprietor: Polymer Group, Inc., North Charleston, SC 29406 (US)
(72) Inventor: DE LEON, Sergio, Diaz, Clayton, NC 27520 (US); CARLSON, Cheryl, Lynn, Willow Springs, NC 27592 (US); CARTER, Nick, Mark, Morresville, NC 28115 (US)
(74) Representative: Schohe, Stefan
(86) International application number: PCT/US2001/021840
(87) International publication number: WO 2002/004729

(56) References cited:
- EP-A- 0 235 309
- EP-A- 0 540 041
- EP-A- 0 577 156
- WO-A-93/20272
- WO-A-94/29505
- WO-A-96/41045
- WO-A-99/63925
- US-A- 5 788 684
- US-A- 5 836 929
- US-A- 6 025 050

## Description

### Technical Field

The present invention relates generally to nonwoven fabrics, and more particularly to a nonwoven fabric which can be configured to include multiple components which are integrated to facilitate liquid-acceptance, liquid-distribution, and liquid-retention, thus facilitating use of the fabric in disposable absorbent articles, such as disposable diapers and feminine hygiene products.

### Background Of The Invention

Disposable absorbent products such as disposable diapers, feminine protection products, incontinence products, and similar articles have met with very widespread acceptance in the marketplace. These types of products have significantly supplanted other types of absorbent articles, such as woven cloth materials, in products such as described above. Development of nonwoven fabric materials, as well as absorbent materials including wood pulp fluff and superabsorbent polymers (SAP) have permitted disposable absorbent products to provide very high levels of absorption and containment, as well as economical and comfortable use by consumers.

A typical disposable absorbent product includes a quantity of absorbent material, ordinarily comprising cellulosic fibrous material such as wood pulp fibers. The use of superabsorbent polymers, ordinarily in particulate form, has been found to desirably enhance liquid-retention. Aside from absorbent material, a typical disposable absorbent article includes an outer, substantially liquid-impermeable layer, and an inner, substantially liquid-permeable layer through which liquids are introduced into the absorbent material. The outer, liquid-impermeable layer acts to contain the liquid within the absorbent structure.

Various attempts have been made to enhance liquid-acceptance and liquid-retention characteristics of disposable absorbent articles. In order to promote liquid-acceptance, a wide variety of different materials have been used for the inner, liquid-permeable layer (sometimes referred to as the "facing" or "top-sheet") of such articles to promote passage of liquid through this layer to the absorbent structure, while avoiding wetting of the layer itself which can detract from comfortable use by consumers. A number of previous constructions have attempted to enhance liquid-retention in disposable absorbent articles by maximizing efficient use of the absorbent structure. Since such absorbent structures may not exhibit a tendency to wick liquid throughout the structure from a point of introduction, so-called liquid-transfer or liquid-distribution layers have been incorporated into absorbent articles to promote use of all portions of the absorbent structure.

As will be appreciated from the above discussion, typical absorbent disposable products include a number of distinct components to achieve fluid management, but efficient manufacture of such products is complicated as the number of distinct components increases. These types of products are typically fabricated from webs of the various constituent layers, with high-speed manufacture requiring efficient handling, registration, and cutting of such webs. It is ordinarily necessary to replenish the supply of each of the constituent webs during manufacture, with an increase in the number of constituent layers requiring a like increase in the periodic replacement of the rolls on which the webs of material are typically stored. Web replenishment can be labor intensive since the rolls themselves may be relatively large and bulky, with each new roll requiring splicing into the end of the previous web. While absorbent structures in such articles may be provided in web-like form, such structures are frequently individually formed from comminuted wood pulp, by air-laying, with the absorbent structures individually cut from a vacuum-formed batt, or individually formed by use of a pocket-forming vacuum apparatus. Again, juxtaposition and registration of each of the absorbent structures with the associated webs of facing and backing materials complicates high-speed manufacture of disposable absorbent articles. Additionally, air-laid absorbent structures typically exhibit very little structural integrity, further complicating high-speed handling after formation.

In view of the foregoing, efforts have been made to provide nonwoven materials, which integrate two or more of the typical layers or components provided in a disposable absorbent structure. However, such efforts have met with only limited success, since integration of the materials from which the various layers are formed can be difficult to effect in an efficient fashion. Additionally, integrated arrangements may diminish effectiveness of one or more of the constituent layers, thus detracting from the efficacy of the resultant absorbent product.

International patent application WO 96/41045 discloses an entangled non-woven composite made from absorbent fibers such as wood pulp fibers and matrix fibers such as polyolefin staple fibers. The absorbent fibers are entangled with the matrix fibers in such a manner so that the composite has absorbent-rich side and a matrix-rich side. Intermediate the two exterior sides there is an entangled interior portion made from a mixture of the absorbent and matrix fibers.

EP 02 35 309 discloses a facing for absorptive articles comprising non-woven fabric having two layers of different fiber-compositions, i.e. a first layer defining a surface to be in contact with the wearer's skin and having a pattern of apertures, and a second layer defined at a rear side with respect to said surface and having no aperture.
A process for making said facing comprises steps of forming said first layer by subjecting fibrous web to high velocity water jet treatment on a support carrying thereon aperture formation elements, forming said second layer by subjecting fibrous web to said treatment or heat fusion treatment, and simultaneously combining said first layer integrally with said second layer with either of said treatments.

EP 0 577 156 discloses composite non-woven web materials and methods of forming the same. The composite non-woven web materials are formed by hydraulically entangling a laminate of (a) at least one layer of meltblown fibers and (b) at least one layer of none-woven material. The non-woven material con comprise at least one of pulp fibers, staple fibers, meltblown fiber and substantially continuous filaments. The non-woven material can also be a net, foam, etc.

International patent application WO 94/29505 discloses a liquid transport material composed of pulp fibers hydraulically needled into a woven fiber structure adapted to have a liquid transport value of at least 12 grams of liquid per gram of material over 30 minutes. The liquid transport material may contain up to about 50 percent, by weight, short staple length fibers as well as effective amount of various particulates. The liquid transport material may be used as a liquid transport component of an absorbent structure which may be part of, for example, a personal care product.

Accordingly, the present invention contemplates a multi-component nonwoven material that acts to efficiently integrate plural components of a disposable absorbent article, thus promoting efficient manufacture, while obtaining the desired level of product performance. A further aspect of the present invention contemplates formation of absorbent structures exhibiting desirably high performance and structural integrity.

### Summary Of The Invention

A multi-component nonwoven fabric embodying the principles of the present invention is particularly suited for use in disposable absorbent articles, and is intended to promote efficient fluid management in such articles, while facilitating economical, high-speed manufacture by reduction in the number of separate components in each article. The fabric is formed with an array of three-dimensional surface projections that minimize contact with a wearer to promote comfort, with the projections further promoting enhanced fluid management. The fabric may optionally be provided with a network of apertures, which are configured to cooperate with the surface projections for control of liquid flow. The fabric includes fibers having liquid acceptance and liquid distribution performance integrated into the layers to promote efficient fluid management, with the fabric optionally including an additional liquid-retention layer. Said aforementioned fibers are provided in layers during nonwoven fabric formation. A further aspect of the present invention contemplates formation of a multi-component nonwoven fabric, by hydroentanglement, which exhibits very high structural integrity without the application of adhesive binder, while providing excellent absorbent capacity.

In accordance with the illustrated embodiment, the present three-dimensional, multi-component nonwoven fabric includes two or more "layers" for receiving, distributing and possibly retaining liquids introduced into the article. These layers may comprise thermoplastic fibers preferentially selected from the group consisting of polyolefins, polyesters, polyamides, and blends thereof, bi-component, splittable thermoplastic fibers of the pie or side-by-side variety, cellulosic fibers such as rayon, wood pulp and blends thereof, and surface profiled fibers such as 4DG. Heat fusible fibers may be introduced into any of the layers to stabilize the structure and enhance the retention of the three-dimensional surface projections of the fabric.

The use of hydroentanglement to effect formation of the present nonwoven fabric permits the fabric to be formed with certain structural features for enhancing versatile use. If desired, a lower layer can be configured to extend into the array of three-dimensional surface projections defined by the upper surface of the fabric. The fabric can be formed with a plurality of apertures that extend from the upper surface through at least a portion of the nonwoven fabric. In a particularly preferred embodiment, the apertures extend from the network of liquid-accepting channels that surround the upstanding projections defined by the upper surface. This arrangement desirably functions such that liquid is channeled and directed by the upstanding projections for passage through the network of apertures defined by the fabric.

As will be appreciated, integration of the desired liquid-acceptance and liquid-distribution properties into one fabric promotes use of the present nonwoven fabric and disposable absorbent article by precluding the need to provide these layers as separate components during product manufacture. A further aspect of the present invention contemplates that the present multi-component fabric includes a lower liquid-retention layer. The liquid retention layer preferably at least partially comprises cellulosic fibers selected from the group consisting of wood pulp fibers and rayon fibers. More preferably, the liquid-retention layer comprises a blend of cellulosic fibers, and thermoplastic fibers having a denier of about 6 and 18, preferentially selected from the group consisting of polyolefins, polyesters, polyamides, and blends thereof. The absorptive capacity of the liquid-retention layer can be enhanced by the provision of superabsorbent polymer within the fibrous structure of the layer.

Absorbent materials formed in accordance with the present invention exhibit desirably high absorbency relative to the thickness of the material, thus permitting disposable products to exhibit the desired degree of absorbency without excessive bulkiness.

Other features and advantages of the present invention will become readily apparent from the following detailed description, the accompanying drawings, and the appended claims.

### Brief Description Of The Drawings

FIGURE 1 is a schematic representation of a production line upon which the process of the present invention is practiced, and the fabric of the present invention is produced;
FIGURES 2A-2C, and FIGURES 3A-3C are diagrammatic plan views of the forming surface of a three-dimensional image transfer device which is used, during hydroentanglement, to practice the present invention; and
FIGURE 4 are illustrations of various three-dimensional image profiles of surface projections of a liquid-acceptance layer of a multi-component nonwoven fabric embodying the principles of the present invention; and
FIGURE 5 are illustrations of various cross-sectional configurations of a nonwoven fabric embodying the principles of the present invention; and
FIGURE 6 is the schematic representation of the nonwoven fabrics of Examples 1 and 2; and
FIGURE 7 is the schematic representation of the nonwoven fabrics of Examples 3 and 4; and
FIGURE 8 is the schematic representation of the nonwoven fabric of Example 5; and
FIGURE 9 is the schematic representation of the nonwoven fabric of Example 6; and
FIGURE 10 is the schematic representation of the nonwoven fabric of Example 8; and
FIGURE 11 is the schematic representation of the nonwoven fabric of Example 10.

### Detailed Description

While the present invention is susceptible of embodiment in various forms, there is shown in the drawings, and will hereinafter be described, presently preferred embodiments, with the understanding that the present disclosure is to be considered as an exemplification of the invention, and is not intended to limit the invention to the specific embodiments illustrated.

The present invention contemplates a multi-component nonwoven fabric that is particularly suited for use in disposable absorbent products, such as disposable diapers, sanitary napkins, incontinence products, and the like. In its multi-component form, the present nonwoven fabric facilitates efficient fluid management in such disposable products, effectively accepting, distributing, and retaining liquid. Formation through hydroentanglement of the multi-component fabric as an integrated structure facilitates its efficient use in high-speed manufacture of disposable absorbent products, since the present fabric can be used in roll form in converting applications. Fabric imaging and patterning, achieved through hydroentanglement on three-dimensional image transfer devices, permits the fabric to be formed with structural features which promote efficient fluid management and wearer comfort. By hydroentanglement of a fibrous structure, including cellulosic fibers such as wood pulp fluff and/or rayon, an absorbent structure can be formed which exhibits a desirably high degree of structural integrity, as well as high absorbent capacity relative to the thickness of the structure.

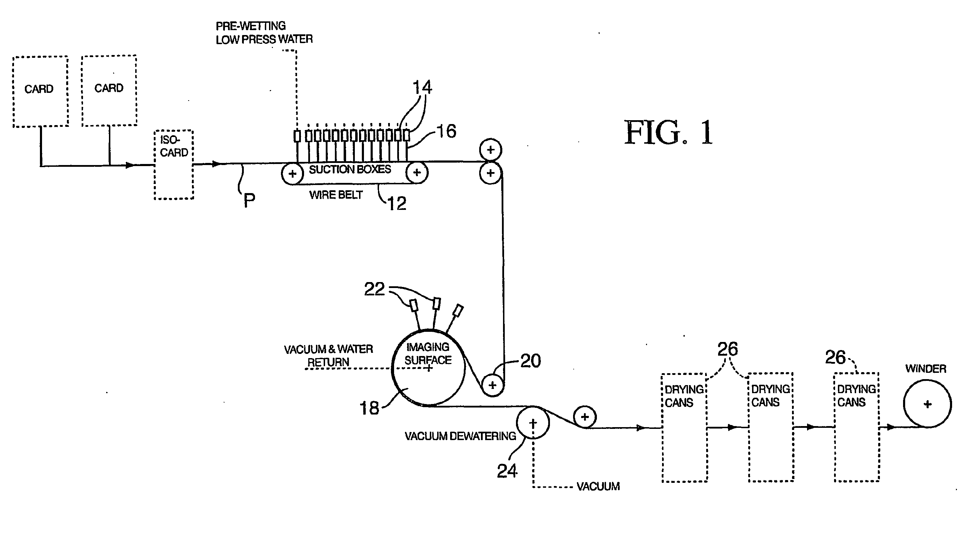

With particular reference to FIGURE 1, therein is illustrated an apparatus for practicing the method of the present invention for forming a nonwoven fabric. The fabric is formed from a fibrous matrix, which comprises fibers selected to promote economical manufacture, while achieving the desired fluid management capabilities for the resultant fabric. The fibrous matrix is preferably carded and subsequently aid-randomized to form a precursor web, designated P.

FIGURE 1 illustrates a hydroentangling apparatus for forming nonwoven fabrics in accordance with the present invention. The apparatus includes a foraminous forming surface in the form of a flat bed entangler 12 upon which the precursor web P is positioned for pre-entangling. Precursor web P is then sequentially passed under entangling manifolds 14, whereby the precursor web is subjected to high-pressure water jets 16. This process is well known to those skilled in the art and is generally taught by U.S. Patent No. 3,485,706, to Evans.

The entangling apparatus of FIGURE 1 further includes an imaging and patterning drum 18 comprising a three-dimensional image transfer device for effecting imaging and patterning of the now-entangled precursor web. After pre-entangling, the precursor web is trained over a guide roller 20 and directed to the image transfer device 18, where a three-dimensional image is imparted into the fabric on the foraminous forming surface of the device. The web of fibers is juxtaposed to the image transfer device 18, and high pressure water from manifolds 22 is directed against the outwardly facing surface from jet spaced radially outwardly of the image transfer device 18. The image transfer device 18, and manifolds 22, may be formed and operated in accordance with the teachings of commonly assigned U.S. Patents No. 5,098,764, No. 5,244,711, No. 5,822,823, and No. 5,827,597. It is presently preferred that the precursor web P be given a three-dimensional image suitable to provide fluid management, as will be further described, to promote use of the present nonwoven fabric in disposable absorbent articles. The entangled fabric can be vacuum dewatered at 24, and dried at an elevated temperature on drying cans 26.

The formation of a multi-component nonwoven fabric embodying the principles of the present invention is effected by forming the precursor fibrous web P to include a first fibrous layer comprising thermoplastic fibers preferentially selected from the group consisting of polyolefins, polyesters, polyamides, and blends thereof; splittable thermoplastic fibers of the pie type; or cellulosic fibers preferentially selected from the group consisting of wood pulp, rayon and blends thereof.

The precursor web further includes a second fibrous layer comprising at least one of the following or a blend thereof; thermoplastic fibers having a denier of about 6 to 18, preferentially selected from the group consisting of polyolefins, polyesters, polyamides, and blends thereof; heat-fusible fibers; profiled thermoplastic fibers; or cellulosic fibers.

Formation of the precursor web P with first and second fibrous layers as described above promotes formation of a multi-component nonwoven fabric having cooperating liquid-acceptance and liquid-distribution layers. It is within the purview of the present invention that a multi-component nonwoven fabric can be formed with a third, liquid-retention layer. To this end, the precursor web P can be formed with a third fibrous layer at least partially comprising cellulosic fibers selected from the group consisting of wood pulp fibers, rayon fibers, and blends thereof. The second fibrous layer of the precursor web is positioned between the first and third fibrous layers for formation of such a three-component fabric.

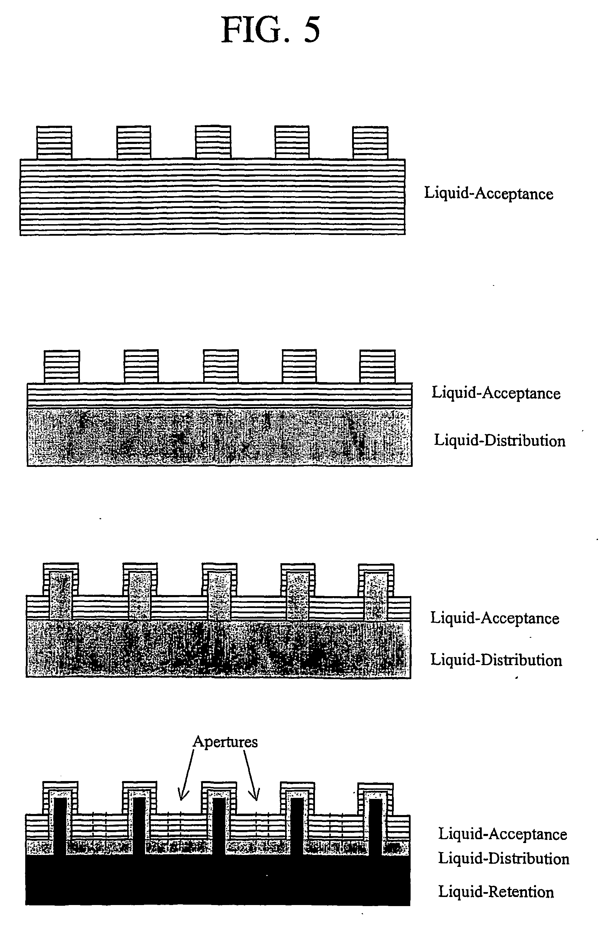

Formation of the present fabric is effected by positioning the precursor web P on the image transfer device 18 with the first fibrous layer of the precursor web positioned adjacent to the foraminous forming surface of the image transfer device. The forming surface of the image transfer device defines an array of surface depressions by which an array of three-dimensional upstanding surface projections is formed on the fabric. The array of upstanding projections defined by the surface of the fabric extends above a network of liquid-accepting channels surrounding the upstanding projections. One or more layers of the nonwoven fabric may extend into the projections defined by the liquid-acceptance layer, as depicted in FIGURE 5. This arrangement desirably acts to channel and control the flow of liquid introduced into the nonwoven fabric, and desirably minimizes the "land" areas of the fabric, which contact the skin of the wearer of a disposable absorbent article. The fluid management capabilities of the present nonwoven fabric can be further enhanced by providing the fabric with a plurality of apertures that preferably extend from the upper surface layer through at least a portion of the nonwoven fabric. Most preferably, these apertures extend from the network of channels that surround the upstanding projections. The apertures cooperate with the upstanding projections to channel and direct liquid into the apertures so that it can be directed into the associated liquid-retaining structure.

When the precursor web P is optionally provided with a third cellulosic fibrous layer, as described above, hydroentanglement of the precursor web results in formation of the present nonwoven fabric. While the liquid retention layer at least partially comprises cellulosic fibers selected from the group consisting of wood pulp fibers, rayon fibers, or blends thereof, the liquid-retention layer may further comprise a blend of such cellulosic fibers, and thermoplastic fibers having a denier between about 6 and 18 preferentially selected from the group consisting of polyolefins, polyesters, polyamides, and blends thereof.

Hydroentanglement of the liquid-retention layer can act to diminish void volumes within the layer that would be expected to reduce the liquid-retaining characteristics of the layer. However, the desired level of liquid-retention can be achieved by selecting fibers that exhibit inherent absorptivity (such as rayon fibers or profiled fibers). Additionally, the liquid-retention layer can be surface napped, at the outwardly facing surface thereof, for enhancing absorbent capacity. Ordinarily, such surface napping can diminish the structural integrity of an absorbent structure. However, because of the relatively high degree of structural integrity imparted to the liquid-retention layer by hydroentanglement in accordance with the present invention, surface napping of the retention layer does not unacceptably diminish its structural integrity.

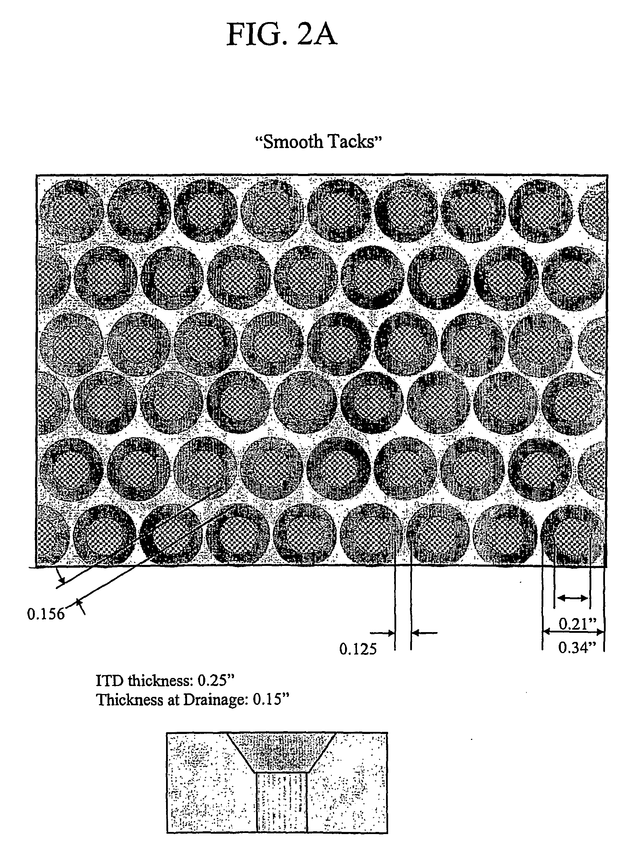

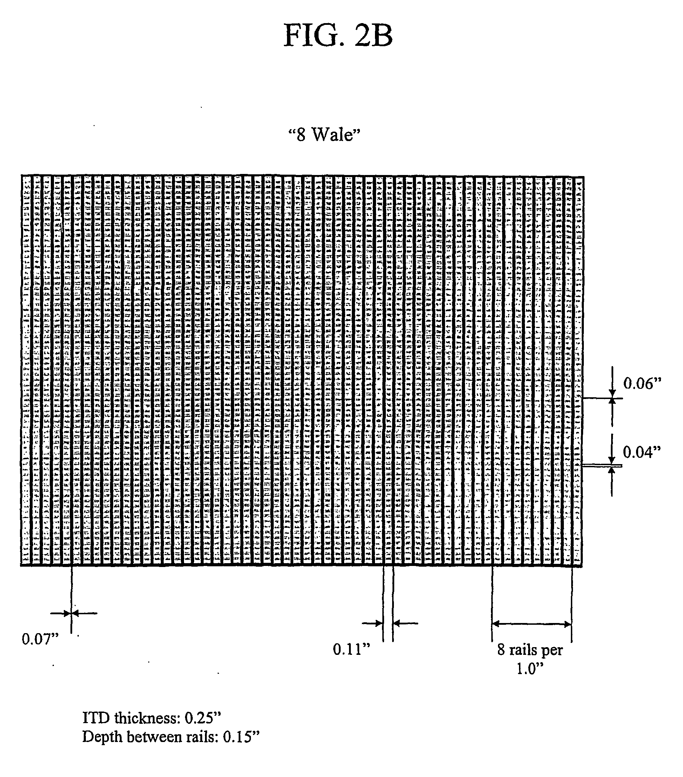

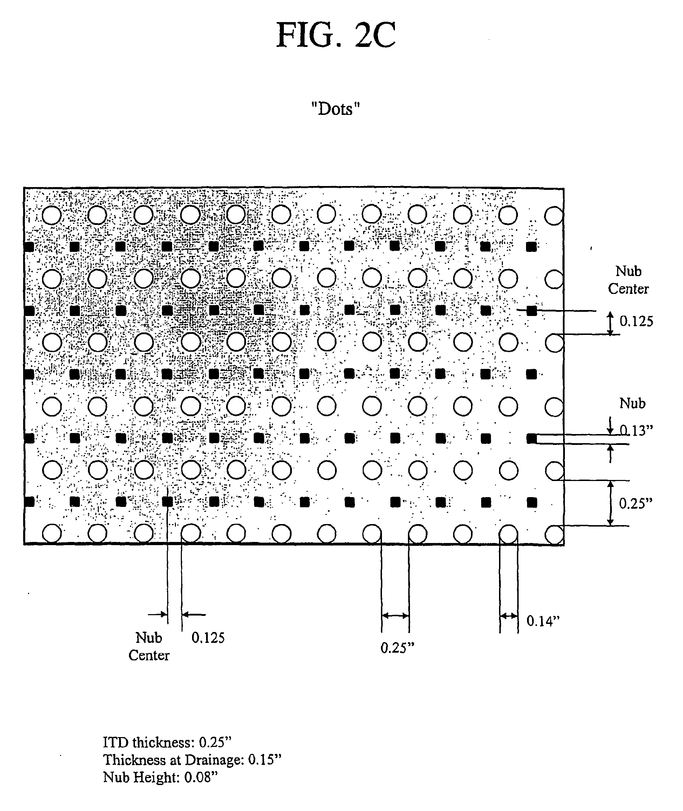

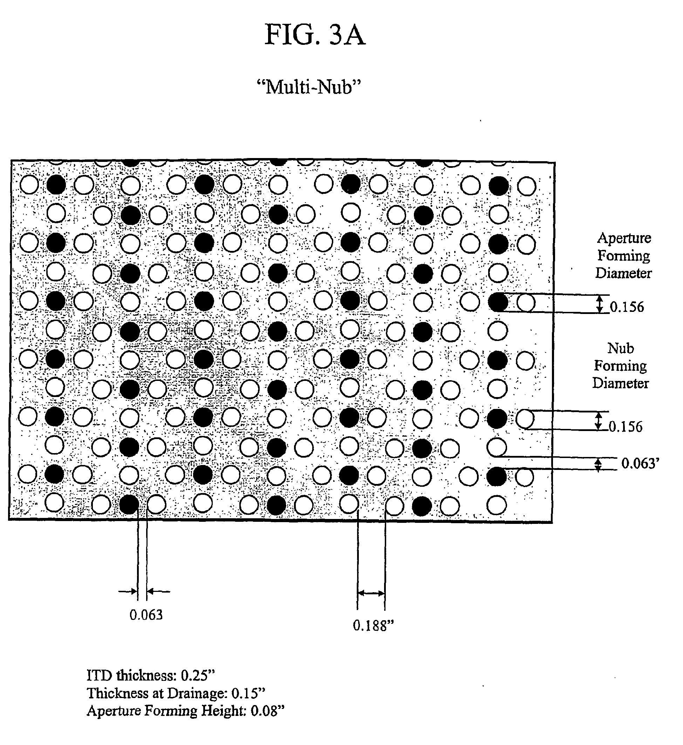

With particular reference to FIGURES 2A-2C and 3A-3C, therein are illustrated various embodiments of the foraminous forming surface of the image transfer device 18 upon which fabrics formed in accordance with the present transfer device 18 upon which fabrics formed in accordance with the present invention are hydroentangled. FIGURE 2A illustrates a forming surface referred to as "smooth tacks", wherein the forming surface comprises an array of surface depressions which taper generally downwardly from an outside diameter of 0,86 cm (0.34 inches) to a lower, for aminous grid-like circular region having a diameter of 0,053 cm (0.021 inches). FIGURE 2B illustrates a forming surface designated "8 wale", having a series of upstanding rails which are positioned above and intersect with a lower series of rails, whereby an array of surface depressions is defined. FIGURE 2C, showing a pattern designated as "dots", shows,a forming surface including an array of depressions (light colored) each having a diameter of 8,35 cm (0.14 inches), spaced 0,64 cm (0.25 inches) apart, with "nubs" (dark colored) extending upwardly from the forming surface for formation of apertures in a nonwoven fabric formed thereon.

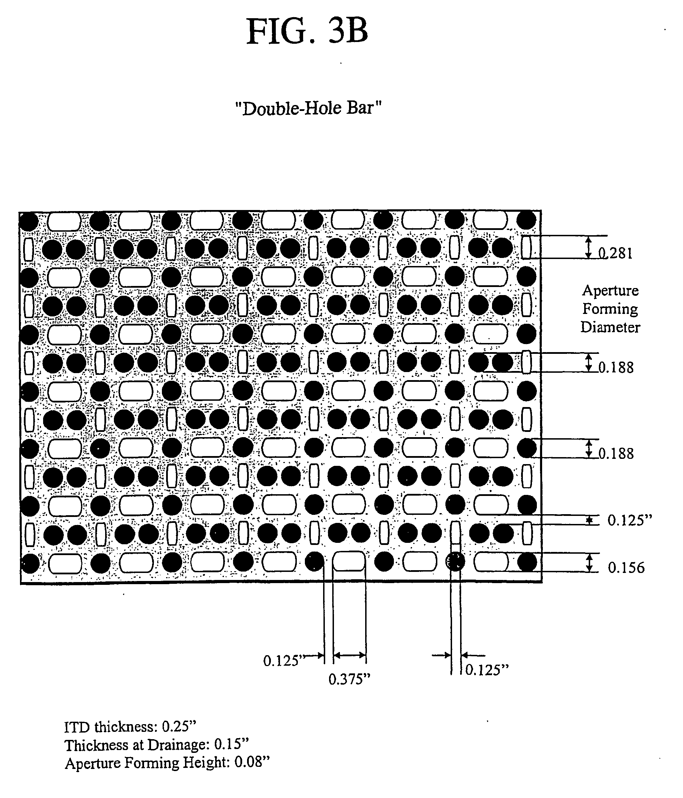

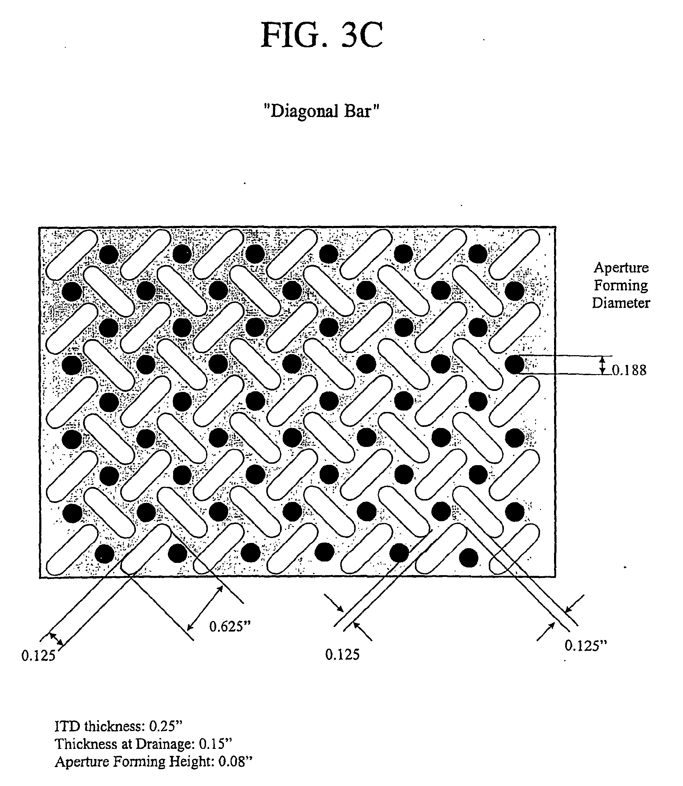

FIGURE 3A shows a forming surface designated "multi-nub" including an array of surface depressions (light colored) each having a diameter of 0.396 cm (0.156 inches) and an array of aperture-forming projections (dark colored). FIGURE 3B illustrates a foraminous forming surface designated "double-hole bar", including an array of generally elongated surface depressions of two different sizes (light colored) and an array of circular, upstanding projections each having a diameter of 0,478 cm (0.188 inches) (dark colored) for formation of apertures in a nonwoven fabric hydroentangled thereon. FIGURE 3C illustrates a foraminous forming surface designated "diagonal bar" wherein an array of elongated surface depressions, each having a length of 1,588 cm (0.625 inches), is arranged with an array of upstanding circular projections each having a diameter of 0,478 cm (0.188 inches) for formation of apertures in a nonwoven fabric formed thereon.

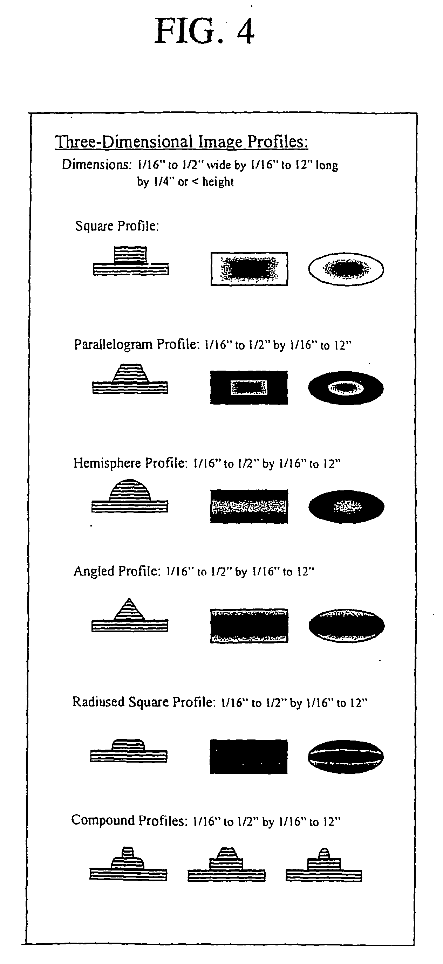

The surface depressions defined by the forming surfaces of the image transfer device used for practicing the present invention can be configured in accordance with the image profiles as shown in FIGURE 4. The profiles can be selected to optimize fluid management, particularly optimizing control and direction of liquid into the network of channels and associated apertures, which surrounds the upstanding projections of the liquid-acceptance layer of the present nonwoven fabric.

In addition to formation of the liquid-retention layer of the present fabric from cellulosic fibers, it is further contemplated that this layer may comprise superabsorbent polymer for enhancing the absorbent capacity of the layer. Superabsorbent polymers are well known in the art, and frequently are employed in particulate form. However, these types of materials are also available in fiber form, and can be heat-activated after fabric formation so that the polymer exhibits the desired superabsorbency. By integration of such fibers into the cellulosic fibrous layer which forms the liquid-retention layer of the present fabric, the fibrous superabsorbent polymer can be heat-activated by drying cans 26 of an apparatus which is illustrated in FIGURE 1.

### EXAMPLE 1

A multi-component fabric is formed with an apparatus such as illustrated in FIGURE 1 by forming a prebond web from carded webs, two of which were air-randomized, comprising 50% by weight 6.0 denier by 5.08 cm (2.0 inch) T-216 PET fibers, and 50% by weight 1.5 denier by 3,99 cm (1.57 inch) staple length Type 8192 rayon, available from Lenzing. The carded webs are integrated on a flat bed entangler 12 by subjecting the prebond web structure to fluid entanglement under 12 successive manifolds, three each operated at 6.89 bar, 20,68 bar, 41,37 bar and 55,16 bar (100 psi, 300 psi, 600 psi, and 800 psi). Each manifold defines orifices of 0,127 mm (0.005-inch) diameter, with 43.3 orifices per 2,54 cm (inch). Line speed was 0.23 m/s (15 yards per minute) with the web subjected to drying at an elevated temperature by three drying cans each operated at 149°C (300° F).

Additional carded web of the same construction as above was layered onto the above-described prebond web, with the resultant precursor web hydroentangled on flat bed entangler 12 by twelve manifolds, three each operated at 6,89 bar, 20,68 bar, 41,37 bar and 68,95 bar (100 psi, 300 psi, 600 psi, and 1000 psi).

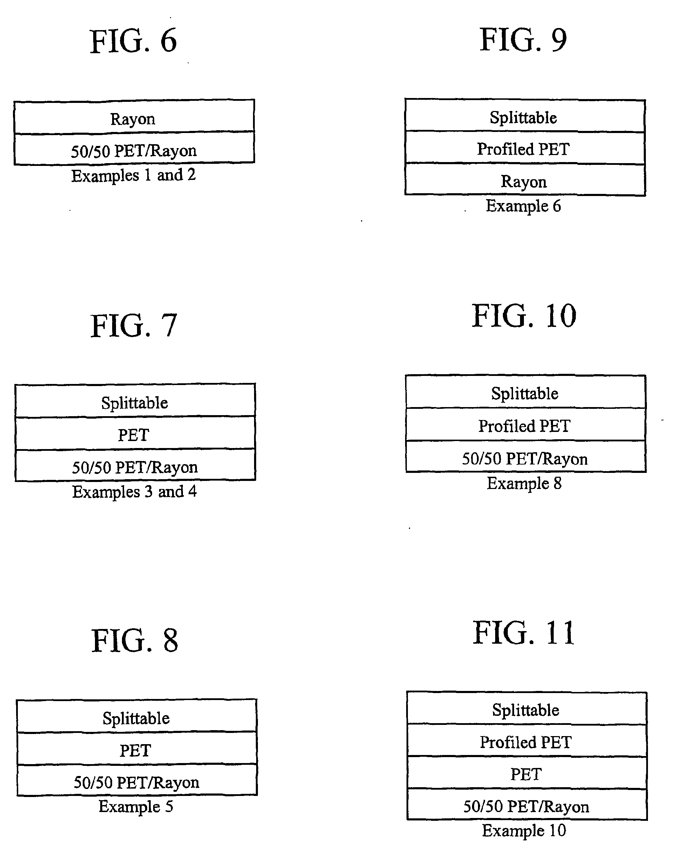

The final fabric was formed by adding four carded webs, two of which were air-randomized, to the above-described ebond web, with each of these fluther card webs comprising 1.5 denier by 3,99 cm (1.57 inch) staple length Type 8192 rayon, with each of these cards being air-randomized. These four, carded webs were layered onto the above-described prebond web, with the resultant precursor web hydroentangled on flat bed entangler 12 by twelve manifolds configured as described above and operated at 6,89 bar, 20,68 bar, 41,37 bar and 68,95 bar (100 psi, 300 psi, 600 psi, and 1000 psi). This precursor web was then imaged and patterned on image transfer device 18 having a "smooth tacks" pattern as illustrated in FIGURE 2A, with three entangling manifolds 22 each operated at 275,8 bar (4000 psi), with each manifold having an orifice strip having orifices of 0,127 mm (0.005 inch) diameter, with 43.3 orifices per 2,54 cm (inch). The fabric was dried at an elevated temperature by three drying cans operate at 149°C at (300° F), with a formation line speed of 0,23 m/s (15 yards per minute), and a final fabric basis weight of 0,17 kg/m² (8.5 ounces per square yard). In the resultant multi-component fabric, the first fibrous layer of the precursor web consisted of all rayon fibers, and were positioned against the forming surface of the image transfer device to form the surface projections, as shown schematically in FIGURES 6. The combined cellulosic layers result in a fabric having a high degree of wickability, and to be particularly useful in the rapid relocation of fluid away from the imaged rayon surface.

### EXAMPLE 2

A multi-component fabric was formed as described above in connection with Example 1, except that the additional carded web comprising 50% by weight 6.0 denier by 5,08 cm (2.0 inch) T-216 PET fibers, and 50% by weight 1.5 denier by 3,99 cm (1.57 inch) staple length Type 8192 rayon, available from Lenzing was not added to the prebond web and an image transfer device having a forming surface configured in accordance with the "8 wale" configuration illustrated in Figures 2B was employed, with three entangling manifolds 22 each operated at 165,5 bar (2400 psi). The resultant multi-component fabric had a basis weight of 0,10 kg/m² (5.1 ounces per square yard).

### EXAMPLE 3

A multi-component fabric is formed with an apparatus such as illustrated in FIGURE 1 by forming prebond web from first and second carded webs each comprising air-randomized 6.0 denier by 5,08 cm (2.0 inch) T-216 PET fibers available from Wellman. Third and fourth carded webs are placed on the first two carded webs, with each of the third and fourth webs comprising 40% by weight 6.0 denier by 5,08 cm (2.0 inch) T-216 PET fibers, and 60% by weight 1.5 denier by 3,99 cm (1.57 inch) staple length Type 8192 rayon, available from Lenzing.

The carded webs are integrated on a flat bed entangler 12 by subjecting the prebond web structure to fluid entanglement under 12 successive manifolds, three each operated at 6,89 bar, 20,68 bar, 41,37 bar and 55,16 bar (100 psi, 300 psi, 600 psi, and 800 psi). Each manifold defines orifices of 0,127 mm (0.005-inch) diameter, with 43.3 orifices per 2,54 cm (inch). Line speed was 0,23 m/s (15 yards per minute), with the web subjected to drying at an elevated temperature by three drying cans each operate at 149°C (300° F). The final basis weight of the prebond was 0,064 kg/m² (3.2 ounces per square yard).

The final fabric was formed by adding two additional carded webs to the above-described prebond web; the first carded web comprising air-randomized 6.0 denier by 5,08 cm (2.0 inch) T-216 PET fiber, randomized and the total weight being 0,026kg/m² (1.3 ounces per square yard), the second carded web comprising 3.0 denier by 5,08 cm (2.0 inch) T-502 splittable fiber (16 sub-denier, PET/nylon, 0.19 denier sub-denier, segmented pie, available from Fiber Innovation Technology, Inc.), with each of these cards being air-randomized and the total weight being 0,026 kg/m² (1.3 ounces per square yard). These carded webs were layered onto the above-described prebond web, with the resultant precursor web hydroentangled on flat bed entangler 12 by twelve manifolds configured and operated as described above. This precursor web was then imaged and patterned on image transfer device 18 having a "smooth tacks" pattern as illustrated in FIGURE 2A, with three entangling manifolds 22 each operated at 196,5 bar (2850 psi), with each manifold having an orifice strip having orifices of 0,127 mm (0.005 inch) diameter, with 43.3 orifices per 2,54 cm (inch). The fabric was dried at an elevated temperature by three drying cans operated a 149°C (300° F), with a formation line speed of 0,23 m/s (15 yards per minute), and a final fabric basis weight of 0,124 kg/m² (6.2 ounces per square yard). In the resultant multi-component fabric, the splittable fibers provided the first fibrous layer of the precursor web, and were positioned against the forming surface of the image transfer device for formation of the liquid-acceptance layer. The two layers of PET fibers formed the second fibrous layer of the precursor web, and formed the liquid-distribution layer of the resultant fabric. The PET/rayon fibers formed the third, cellulosic fibrous layer of the precursor web, and formed the liquid-retention layer of the resultant fabric.

### EXAMPLE 4

A multi-component fabric was formed as described above in connection with Example 3, except that an image transfer device having a forming surface configured in accordance with the "dots" configuration illustrated in FIGURE 2C was employed. The resultant multi-component fabric had a basis weight of 0,124 kg/m² (6.2 ounces per square yard).

### EXAMPLE 5

A prebond web was formed from four carded, air-randomized webs, two of which were air-randomized, each comprising 50% by weight 11.0 denier by 5,08 cm (2.0 inch) T-261 PET fibers from Wellman, and 50% by weight 1.5 denier by 3,99 cm (1.57 inch) staple Type 8191 from Lenzing. The webs were hydroentangled on the flat bed entangler 12 by twelve manifolds 0,127 mm (0.005-inch) orifices by 43.3 orifices per 2,54 cm (inch), with three each operated at 6,89 bar, 20,68 bar, 41,37 bar, and 41,37 bar (100 psi, 300 psi, 600 psi, and 600 psi). Three drying cans were operated at 149°C (300° F), with a line speed of 0,31 m/s (20 yards per minute). The resultant prebond web had a basis weight of 0,07 kg/m² (3.5 ounces per square yard).

The final nonwoven fabric embodying the present invention was formed by adding two additional carded webs, the first carded web comprising 6.0 denier by 5,08 (2.0 inch) T-216 PET fibers, and the second carded web comprising air-randomized 2.5 denier by 5.08 cm (2.0 inch) N-91 splittable fiber (20 sub-denier, segmented pie, PET/nylon, 0.12 denier sub-denier fibers from Unitika). These carded webs were layered onto the above-described prebond web, and hydroentangled therewith on the flat bed entangler 12 with orifice strips as described above, with three each of the twelve manifolds successively operated at 6,89 bar, 20,68 bar, 41,37 bar and 75,85 bar (100 psi, 300 psi, 600 psi, and 1100 psi).

The precursor web was then positioned on the image transfer device 18, having the "smooth tacks" forming surface illustrated in FIGURE 2A. The precursor web was imaged and patterned by operation of three manifolds 22 at 171,0 bar 2480 psi, with each manifold including 0,127 mm (0.005-inch) diameter orifices, 43.3 orifices per 2,54 cm (inch). The splittable fibers were positioned on the forming surface of the image transfer device, and provided the first fibrous layer of the precursor web. The 6 denier PET fibers provided the second fibrous layer of the precursor web, with the PET/rayon (large denier) fibers providing the third, cellulosic fibrous layer of the precursor web. The resultant nonwoven fabric, having liquid-acceptance, liquid-distribution, and liquid-retention layers, was dried at an elevated temperature by drying cans 26 operated at 149°C (300° F) (two cans), with a line speed of 0,155 m/s (10 yards per minute). Final fabric basis weight was 0,14 kg/m² (7.0 ounces per square yard).

### EXAMPLE 6

A prebond web was formed from three carded webs, two of which were air-randomized, each comprising 50% by weight, 1.5 denier by 3,99 cm (1.57 inch) staple 8192 rayon, and 50% by weight, 1.5 denier by 3,99 mm (1.57 inch) staple 8191 rayon. The card webs were hydroentangled on flat bed entangler 12 with manifolds configured as described above, with three each of the manifolds operated at 3,45 bar, 20,68 bar and 27,56 bar (50 psi, 300 psi, and 400 psi). The prebond web was dried on two drying cans operated at 149°C at (300° F), with a line speed of 0,31 m/s (20 yards per minute), and a final basis weight of 68,7 g/m² of (2.0 ounces per square yard).

A second prebond web was formed from 3 carded webs, two of which were air-randomized, each comprising 3.0 denier by 1.5 inch staple 4DG fiber available from Fiber Innovation Technology, Inc., a profiled fiber configured in accordance with U.S. Patent No. 5,855,798 and 5,977,429. The card webs were hydroentangled on flat bed entangler 12 with manifolds configured as described above, with three each of the manifolds operated at 3,45 bar, 20,68 bar and 27,56 bar (50 psi, 300 psi, and 400 psi). The prebond web was dried on two drying cans operated at 282°C (300°F), with a line speed of 0,31 m/s (20 yards per minute), and a final basis weight of 0,03 kg/m² (1.5 ounces per square yard).

A third prebond web was formed from 3 carded webs, two of which were air-randomized, with each of the carded webs comprising 3.0 denier by 5,08 cm (2.0 inch) staple T-502 splittable fiber (16 sub-denier, PET/nylon, 0.19 denier sub-denier, segmented pie, from Fiber Innovation Technology, Inc.). The carded webs were hydroentangled on the flat bed entangler, having manifolds as described above, with three each of the manifolds operated at 3,45 bar, 20,68 bar, 41,37 bar and 55,16 bar (50 psi, 300 psi, 600 psi, and 800 psi). The web was positioned on a 100-mesh screen, and subjected to the action of water jets (three manifolds, 0,127 mm (0.005 inch) diameter orifices, 43.3 orifices per 2,54 cm (inch), operated at 137,9 bar (2500 psi), to effect splitting of the splittable fibers. The prebond web was dried on two drying cans operated at 149°C at (300° F), with a line speed of 0,30 m/s (20 yard per minute), and a final basis weight of 0,037 kg/m² (1.83 ounces per square yard).

The three prebond fabrics described above were layered in the following given above, and were pre-entangled on the flat bed entangler 12 as described above, with each of three manifolds operated at 3,75 bar, 20,68 bar, 41,37 bar and 55,16 bar (50 psi, 300 psi, 600 psi, and 800 psi). This precursor web was then positioned on the image transfer device 18 having a forming surface of "dots" configured in accordance with FIGURE 2C, with each of the three manifolds 22 operated at 110,3 bar (1600 psi). The splittable fibers were positioned adjacent the image transfer device, with the 3 denier 4DG PET fibers positioned in the next layer, and with the rayon fibers positioned in the next layer. The fabric was dried at an elevated temperature on three drying cans operated at 149°C (300° F), with the fabric formed At a line speed of 0,23 m/s (15 yards per minute). Final fabric basis weight was 0,122 kg/m² (6.05 ounces per square yard).

### EXAMPLE 7

A material was formed as described in Example 6. Application of a teasel brush was employed to the rayon-containing side of the material until the material reached the bulk shown in Table 3.

### EXAMPLE 8

A multi-component fabric is formed as described in Example 6 except the first prebond web was formed from four carded webs, two of which were air-randomized, each comprising 50% by weight 11.0 denier by 5,08 cm (2.0 inch) T-261 PET fibers from Wellman, and 50% by weight 1.5 denier by 3,99 cm (1.57 inch) rayon staple Type 8191 from Lenzing. The resultant prebond web had a basis weight of 0,071 kg/m² (3.5 ounces per square yard). The final fabric basis weight was 0,141 kg/m² (6.98 ounces per square yard).

### EXAMPLE 9

A material was formed as described in Example 8. Application of a teasel brush was employed to the rayon-containing side of the material until the material reached the bulk shown in Table 3.

### EXAMPLE 10

A multi-component fabric is formed as described in Example 6 except the first prebond web was formed from two carded webs each comprising air-randomized 6.0 denier by 5,08 cm (2.0 inch) T-216 PET fibers available from Wellman. Third and fourth carded webs are placed on the first two carded webs, with each of the third and fourth webs comprising 50% by weight 6.0 denier T-216 PET fibers, and 50% by weight 1.5 denier by 3,99 cm (1.57 inch) staple length Type 8192 rayon, available from Lenzing. The line speed was 0,23 m/s (15 yards per minute), with the web subjected to drying at an elevated temperature by three drying cans each operated at 149°C (300° F). The final basis weight of the prebond was 0,064 kg/m² (3.2 ounces per square yard). The final fabric basis weight was 0,134 kg/m² (6.67 ounces per square yard).

### EXAMPLE 11

A material was formed as described in Example 10. Application of a teasel brush was employed to the rayon-containing side of the material until the material reached the bulk shown in Table 3.

Accompanying Tables 1, 2, and 3, set forth test data obtained in connection with testing of the above-described Examples. Testing was done in accordance with the following standard test methods.

| **Test** | **Method** |
|---|---|
| Basis Weight (*oz*/*sy*) | ASTM D3776 |
| Thickness (mils | ASTM D5729 |
| Tensiles - MD and CD Grab (lb/in) | ASTM D5034 |
| Elongation - MD and CD Grab (%) | ASTM D5034 |
| Absorbency - Time (sec) | ASTM D1117 |
| Absorbency - Capacity (%) | ASTM D1117 |
| Strike Through (sec) | EDANA 150.3 |
| Rewet (grams | EDANA 151.1 |

No Load Capacity (gr. Liquid / gr. Fabric): This test entails the following procedure. A 12.7 cm by 12,7 cm (5 inch by 5 inch) sample is weighed. Then it is submerged into a saline solution (0.9%) for 15 minutes. The sample is removed from the solution and allowed to drain vertically for one minute. The sample is then weighed again. The no load capacity is reported as the ratio of the fabric weight after submersion to the weight of fabric before submersion.

For comparison purposes, a disposable "swimmer" absorbent product (i.e., a disposable absorbent swimwear article) was tested, with results as shown. As will be observed from the test data, fabrics formed in accordance with the present invention provided excellent absorbent characteristics, particularly by comparison of the absorbency of the fabrics (as a percentage of fabric weight), relative to the thickness of the fabrics. Additionally, fabrics formed in accordance with the present invention exhibited excellent structural integrity especially compared to the disposable "swimmer" absorbent product, thus facilitating their efficient use in high-speed manufacture of disposable absorbent products.

Exemplary disposable absorbent articles which can be configured in accordance with the present invention include disposable diapers, disposable training pants and "swimmers", sanitary protection products, adult incontinent products, incontinence pads, and like disposable absorbent structures. U.S. Patents No. 4,695,278, to Lawson, and No. 4,704,116, to Enloe, illustrate exemplary disposable diapers. U.S. Patents No. 4,938,753, No. 4,938,757, and No. 4,940,464, all to Van Gompel, illustrate exemplary disposable training pants. U.S. Patents No. 4,589,876, and No. 4,687,478, both to Van Tilburg, illustrate exemplary sanitary napkin constructions.

**TABLE 1**

| | | Example 1 | | Example 2 | |
|---|---|---|---|---|---|
| | "Target" | Face Up | Face Down | Face Up | Face Down |
| TEST | | | | | |
| Fiber Composition | Consumer Product | Rayon Face 50% PET 50%Rayon Back | | Rayon Face 50% PET 50%Rayon Back | |
| Image | | Tacks | | 8 Wales | |
| Basis Weight (oz/sy) | 10.45 | 8.5 | | 6.3 | |
| (kg/cm²) | 0,211 | 0,171 | | 0,127 | |
| Thickness (mils) | 1457.5 | 90 | | 60 | |
| Tensiles - MD Grab (1b/in) | 2.2 | 113.85 | | 65.79 | |
| (kg/m) | 39,29 | 2033,13 | | 1174,88 | |
| Tensiles-CD Grab (1b/in) | | 444.8 | | 30.86 | |
| (kg/m) | | 800,04 | | 551,10 | |
| Elongation -MD Grab (%) | 21.67 | 43.29 | | 47.37 | |
| Elongation - GD Grab (%) | | 101.96 | | 87.9 | |
| Absorbency-Time (sec) | 7 | 1.1 | | 1.2 | |
| Absorbency - Capacity (%) | 1082 | 609 | | 710 | |
| No Load Capacity (gr. Liquid / gr. Fabric) | 9.25 | 6.25 | | 5.95 | |
| Strike Through (sec) | 1.45 | 0.28 | 0.57 | 0.69 | 0.88 |
| Rewet (grams) | 2.74 | 3.53 | 3.23 | 3.61 | 3.37 |

**TABLE 2.**

| | | Example 3 | Example 4 | Example 5 |
|---|---|---|---|---|
| | "Target" | Face Up | Face Up | Face Up |
| TEST | | | | |
| Fiber Composition | Consumer Product | Splittable Face 6dpf PET 6dpf - Rayon Back | Splittable Face 6dpf PET 6dpf - Rayon Back | Splittable Face 6dpf PET 11 dpf PET/ Rayon Back |
| Image | | Tacks | Dots | Dots |
| Basis Weight (oz/sy) | 10.45 | 6.2 | 6.24 | 7.04 |
| (kg/m²) | 0,211 | 0,125 | 0,126 | 0,142 |
| Thickness (mils) | 1457.5 | 64.5 | 71 | 85 |
| Tensiles MD Grab (1b/in) | 2.2 | 109.77 | 118.72 | 106.61 |
| (kg/m) | 39,29 | 1960,27 | 2120,10 | 1903,84 |
| Tensiles - CD Grab (1b/in) | | 54.15 | 57.46 | 62.23 |
| (kg/m) | | 967,00 | 1026,12 | 1111,30 |
| Elongation - MD Grab (%) | 21.67 | 55.09 | 53.08 | 56.72 |
| Elongation - CD Grab (%) | | 117.45 | 10233 | 87.7 |
| Absorbency - Time (sec) | 7 | 2.9 | 4 | 2 |
| Absorbency - Capacity (%) | 1082 | 549 | 516 | 617 |
| No Load Capacity (gr. Liquid / gr. Fabric) | 9.25 | 4.35 | 4.37 | 4.72 |
| Strike Through (sec) | 1.45 | 0.98 | 0.81 | 0.52 |
| Rewet (grams) | 2.74 | 3.64 | 3.65 | 2.51 |

**TABLE 3**

| | Example 6 | Example 7 | Example 8 | Example 9 | Example 10 | Example 11 |
|---|---|---|---|---|---|---|
| | | Hand Napped Back Side | | Hand Napped Back Side | | Hand Napped Back Side |
| TEST | | | | | | |
| Fiber Composition Face | Splittable 4DG PET Rayon | Splittable 4DG PET Rayon | Splittable 4DG PET 11 dpf PET/ Rayon | Splittable 4DG PET 11 dpf PET/ Rayon | Splittable 4DG PET 6 dpf PET/ Rayon 6 dpf PET | Splittable 4DG PET 6 dpf PET/ Rayon 6 dpf PET |
| Image | Dots | Dots | Dots | Dots | Dots | Dots |
| Basis Weight (oz/sy) | 6.05 | 6.35 | 6.98 | 6.91 | 6.67 | 6.72 |
| (kg/m²) | 0,122 | 0,128 | 0,141 | 0,140 | 0,134 | 0,136 |
| Thickness (mils) | 63 | 85 | 61 | 93 | 81 | 104 |
| Tensiles - MD Grab (lb/in) | 41.8 | 37.19 | 58.7 | 44.58 | 40.98 | 40.69 |
| (kg/m) | 746,46 | 664,14 | 1048,26 | 796,11 | 731,82 | 726,64 |
| Elongation- MD Grab (%) | 26.29 | 34.05 | 33.57 | 29.82 | 35.75 | 37.8 |
| Absorbency - Time (sec) | 3 | 8 | 1.5 | 4.5 | 20 | 30 |
| Absorbency - Capacity (%) | 751 | 963 | 607 | 899 | 911 | 1158 |

## Claims

1. A multi-component nonwoven fabric for use in disposable absorbent articles, said fabric comprising:
an upper, liquid-acceptance layer having an upper surface for receiving liquids introduced into said absorbent article, said upper layer comprising fibers selected from thermoplastic polymers, splittable thermoplastic fibers, or cellulosic fibers,
said upper surface of upper layer defining an array of upstanding projections extending above a network of liquid-accepting channels surrounding said upstanding projections; and
a next liquid-distribution layer juxtaposed to said upper layer, and hydroentangled therewith in liquid-transferring relationship for receiving liquid from said upper layer for distribution to said absorbent article,
said next layer comprising a blend of fibers including: (1) fibers selected from the group consisting of thermoplastic polymers, (2) profiled thermoplastic fibers, or (3) cellulosic fibers.

2. A multi-component nonwoven fabric in accordance with claim 1, including:
a lower liquid-retention layer juxtaposed to said liquid-distribution layer on the side thereof opposite said liquid-acceptance layer, said liquid-retention layer being hydroentangled in liquid-transferring relationship with said liquiddistribution layer, said liquid-retention layer at least partially comprising cellulosic fibers selected from the group consisting of wood pulp fibers, rayon fibers, and blends thereof.

3. A multi-component nonwoven fabric in accordance with claim 2, wherein:
said liquid-retention layer comprises a blend of said cellulosic fibers and thermoplastic polymer fibers having a denier of between about 6 and 18.

4. A multi-component nonwoven fabric in accordance with claim 3, wherein:
thermoplastic polymers are selected from the group consisting of polyolefins, polyesters, polyamides, and blends thereof.

5. A multi-component nonwoven fabric in accordance with claim 4, wherein:
polyolefins are selected from the group consisting of polypropylene, polyethylene, and blends thereof.

6. A multi-component nonwoven fabric in accordance with claim 4, wherein:
polyesters are selected from the group consisting of polyethylene terephthalate, polybutylene terephthalate, and blends thereof.

7. A multi-component nonwoven fabric in accordance with claim 4, wherein:
polyamide include nylon.

8. A multi-component nonwoven fabric in accordance with claim 2, wherein:
said next layer comprises a blend of fibers including: (1) fibers having a denier of about 6 to 18 selected from the group consisting of thermoplastic polymers, (2) profiled thermoplastic fibers, or (3) cellulosic fibers.

9. A multi-component nonwoven fabric in accordance with claim 1, wherein:
said liquid-distribution layer extends into said array of projections defined by the upper surface of said liquid-acceptance layer.

10. A multi-component nonwoven fabric in accordance with claim 2, wherein:
a lower surface of said liquid-retention layer, opposite said liquiddistribution layer, is surface-napped for enhanced loft.

11. A multi-component nonwoven fabric in accordance with claim 1, wherein:
said fabric defines a plurality of apertures extending from said network of liquid-accepting channels through said lower liquid-distribution layer.

12. A method of making a multi-component nonwoven fabric, comprising the steps of:
forming a layered precursor fibrous web including a first fibrous layer comprising fibers selected from the group consisting of polypropylene, polyester, polyethylene terephthalate, and nylon and having a basis weight between about 0,010 and 0,030 kg/m² (0.5 and 1.5 ounces/yd²), and a second fibrous layer comprising a blend of fibers including (1) fibers having a denier of about 6 to 18 selected from the group consisting of polyolefins, polyesters, and polyamides, and (2) heat-fusible fiber, said second layer having a basis weight between about 0,010 and 0,020 kg/m² (0.5 and 1.0 ounces/yd²);
providing a three-dimensional image transfer device having a for aminous forming surface defining an array of surface depressions;
positioning said precursor fibrous web on said image transfer device with said first fibrous layer positioned adjacent said foraminous forming surface, and
hydroentangling said precursor web whereby said precursor web is imaged and patterned on said image transfer device, to thereby form a multicomponent nonwoven fabric, said first fibrous layer forming a liquid-acceptance layer of said fabric having an array of upstanding projections extending above a network of liquid-accepting channels, said array of projections corresponding to said array of surface depressions defined by said for aminous forming surface, said second fibrous layer forming a liquid-distribution layer of said fabric juxtaposed to said liquid-acceptance layer and hydroentangled therewith in liquid-transferring relationship.

13. A method of making a multi-component nonwoven fabric in accordance with claim 12, including:
forming said precursor web with a third fibrous layer at least partially comprising cellulosic fibers selected from the group consisting of wood pulp fibers, rayon fibers, and blends thereof, said second fibrous layer being positioned between said first and third fibrous layers,
said hydroentangling step including forming said third fibrous layer as a liquid-retention layer of said fabric.

14. A method of making a multi-component nonwoven fabric in accordance with claim 13, including:
forming said third fibrous layer from a blend of said cellulosic fibers and fibers having a denier about 6 and 18 selected from the group consisting of polyesters, polyolefins, and polyamides fibers.

15. A method of making a multi-component nonwoven fabric in accordance with claim 13, including:
forming said liquid-acceptance layer with superabsorbent polymer therein.

16. A method of making a multi-component nonwoven fabric in accordance with claim 15, including:
providing said superabsorbent polymer in a fibrous form in said third fibrous layer of said precursor web, and
drying said nonwoven fabric at an elevated temperature to activate said superabsorbent polymer.

17. A method of making a multi-component nonwoven fabric in accordance with claim 13, including:
surface napping said liquid-retention layer.

18. A method of making a multi-component nonwoven fabric in accordance with claim 12, including:
forming a plurality of apertures extending from said network of liquidaccepting channels through said liquid-acceptance and liquid-distribution layers.

## Patentansprüche

1. Mehrkomponenten-Vliesstoff zur Verwendung in absorbierenden Wegwerfartikeln,
wobei der Vliesstoff umfasst:
eine obere Flüssigkeitsannahmeschicht mit einer oberen Oberfläche zum Empfangen von Flüssigkeiten, die in den absorbierenden Artikel eingeführt werden, wobei die obere Schicht Fasern aufweist, die aus thermoplastischen Polymeren, splitbaren thermoplastischen Fasern oder Zellulosefasern ausgewählt sind,
wobei die obere Oberfläche der oberen Schicht eine Anordnung von aufrechten Vorsprüngen definiert, die sich oberhalb eines Netzwerks von Flüssigkeit annehmenden Kanälen erstrecken, welche die aufrechten Vorsprünge umgeben, und
eine nächste Flüssigkeitsverteilungsschicht, welche neben der oberen Schicht liegt und mit dieser in einer Flüssigkeit übertragenden Beziehung hydroverwickelt ist, um Flüssigkeit von der oberen Schicht zur Verteilung auf den absorbierenden Artikel zu empfangen,
wobei diese nächste Schicht eine Mischung von Fasern umfasst, welche enthält:
(1) Fasern, die aus der aus thermoplastischen Polymeren bestehenden Gruppe gewählt sind,
(2) thermoplastische Profilfasern oder
(3) Zellulosefasern.

2. Mehrkomponenten-Vliesstoff gemäß Anspruch 1, welcher umfasst:
eine untere Flüssigkeitsrückhalteschicht, die neben der Flüssigkeitsverteilungsschicht auf derjenigen ihrer Seiten liegt, welche gegenüberliegend zu der Flüssigkeitsannahmeschicht ist, wobei die Flüssigkeitsrückhalteschicht mit der Flüssigkeitsverteilungsschicht in einer Flüssigkeit übertragenden Beziehung hydroverwickelt ist, wobei die Flüssigkeitsrückhalteschicht zumindest teilweise Zellulosefasern aufweist, die aus der Gruppe ausgewählt sind, die aus Holzschlifffasern, Reyonfasern und Mischungen hiervon besteht.

3. Mehrkomponenten-Vliesstoff gemäß Anspruch 2, wobei die Flüssigkeitsrückhalteschicht eine Mischung aus den Zellulosefasern und thermoplastischen Polymerfasern mit einem Denier zwischen ungefähr 6 und 18 umfasst.

4. Mehrkomponenten-Vliesstoff gemäß Anspruch 3, wobei thermoplastische Polymere aus der Gruppe gewählt sind, die aus Polyolefinen, Polyestern, Polyamiden und Mischungen hiervon besteht.

5. Mehrkomponenten-Vliesstoff gemäß Anspruch 4, wobei Polyolefine aus der Gruppe gewählt sind, die aus Polypropylen, Polyethylen und Mischungen hiervon besteht.

6. Mehrkomponenten-Vliesstoff gemäß Anspruch 4, wobei Polyester aus der Gruppe gewählt sind, die aus Polyethylenterephthalat, Polybutylenterephthalat und Mischungen hiervon besteht.

7. Mehrkomponenten-Vliesstoff gemäß Anspruch 4, wobei Polyamid Nylon umfasst.

8. Mehrkomponenten-Vliesstoff gemäß Anspruch 2, wobei die nächste Schicht eine Mischung von Fasern aufweist, die enthält:
(1) Fasern mit einem Denier von ungefähr 6 bis 18, die aus der Gruppe ausgewählt sind, die aus thermoplastischen Polymeren besteht,
(2) thermoplastische Profilfasern oder
(3) Zellulosefasern.

9. Mehrkomponenten-Vliesstoff gemäß Anspruch 1, wobei die Flüssigkeitsverteilungsschicht sich in die Anordnung von Vorsprüngen hinein erstreckt, welche durch die obere Oberfläche der Flüssigkeitsannahmeschicht definiert wird.

10. Mehrkomponenten-Vliesstoff gemäß Anspruch 2, wobei eine untere Oberfläche der Flüssigkeitsrückhalteschicht gegenüber der Flüssigkeitsverteilungsschicht für eine größere Lockerheit an der Oberfläche gekräuselt ist.

11. Mehrkomponenten-Vliesstoff gemäß Anspruch 1, wobei der Stoff mehrere Öffnungen definiert, die sich von dem Netzwerk aus Flüssigkeit aufnehmenden Kanälen durch die untere Flüssigkeitsverteilungsschicht erstrecken.

12. Verfahren zum Herstellen eines Mehrkomponenten-Vliesstoffs, welches die folgenden Schritte umfasst:
Bilden eines geschichteten Vorläufer-Faservlieses, welches eine erste Faserschicht, die Fasern aufweist, die aus der Gruppe gewählt sind, die aus Polypropylen, Polyester, Polyethylenterephthalat und Nylon besteht, mit einem Basisgewicht zwischen ungefähr 0,010 kg/m² und 0,030 kg/m² (0,5 ounces/yd² und 1,5 ounces/yd²), und eine zweite Faserschicht enthält, welche eine Mischung von Fasern aufweist, die (1) Fasern mit einem Denier von ungefähr 6 bis 18, die aus der Gruppe gewählt sind, die aus Polyolefinen, Polyestern und Polyamiden besteht, und (2) unter Wärme schmelzfähige Faser enthält, wobei die zweite Schicht ein Basisgewicht zwischen ungefähr 0,010 kg/m² und 0,020 kg/m² (0,5 ounces/yd² und 1,0 ounces/yd²) aufweist,
Bereitstellen einer dreidimensionalen Bildübertragungsvorrichtung mit einer mit Löchern versehenen Formoberfläche, die eine Anordnung von Oberflächenvertiefungen definiert,
Positionieren des Vorläufer-Faservlieses auf der Bildübertragungsvorrichtung, wobei die erste Faserschicht an die mit Löchern versehene Formoberfläche angrenzend positioniert wird, und
Hydroverwickeln des Vorläufervlieses, wodurch das Vorläufervlies auf der Bildübertragungsvorrichtung mit einem Bild versehen und gemustert wird, um **dadurch** einen Mehrkomponenten-Vliesstoff zu bilden, wobei die erste Faserschicht eine Flüssigkeitsannahmeschicht des Stoffs mit einer Anordnung von aufrechten Vorsprüngen aufweist, die sich oberhalb eines Netzwerks von Flüssigkeit aufnehmenden Kanälen erstrecken, wobei die Anordnung von Vorsprüngen der Anordnung von Oberflächenvertiefungen entspricht, die durch die mit Löchern versehene Formoberfläche definiert wird, wobei die zweite Faserschicht eine Flüssigkeitsverteilungsschicht des Stoffs bildet, die neben der Flüssigkeitsannahmeschicht liegt und mit dieser in einer Flüssigkeit übertragenden Beziehung hydroverwickelt ist.

13. Verfahren zum Herstellen eines Mehrkomponenten-Vliesstoffs gemäß Anspruch 12, welches umfasst:
Bilden des Vorläufervlieses mit einer dritten Faserschicht, die zumindest teilweise Zellulosefasern aufweist, die aus der Gruppe ausgewählt sind, die aus Holzschlifffasern, Reyonfasern und Mischungen hiervon besteht, wobei die zweite Faserschicht zwischen der ersten und dritten Faserschicht angeordnet ist,
wobei der Hydroverwicklungsschritt das Ausbilden der dritten Faserschicht als eine Flüssigkeitsrückhalteschicht des Stoffes umfasst.

14. Verfahren zum Herstellen eines Mehrkomponenten-Vliesstoffs gemäß Anspruch 13, welches das Ausbilden der dritten Faserschicht aus einer Mischung aus den Zellulosefasern und Fasern mit einem Denier von ungefähr 6 und 18 umfasst, die aus der Gruppe ausgewählt sind, die aus Polyester-, Polyolefin- und Polyamidfasern besteht.

15. Verfahren zum Herstellen eines Mehrkomponenten-Vliesstoffs gemäß Anspruch 13, welches das Ausbilden der Flüssigkeitsannahmeschicht mit einem superabsorbierenden Polymer darin umfasst.

16. Verfahren zum Herstellen eines Mehrkomponenten-Vliesstoffs gemäß Anspruch 15, welches umfasst:
Bereitstellen des superabsorbierenden Polymers in einer Faserform in der dritten Faserschicht des Vorläufervlieses und
Trocknen des Vliesstoffs bei einer erhöhten Temperatur, um das superabsorbierende Polymer zu aktivieren.

17. Verfahren zum Herstellen eines Mehrkomponenten-Vliesstoffs gemäß Anspruch 13, welches das Oberflächenkräuseln der Flüssigkeitsrückhalteschicht umfasst.

18. Verfahren zum Herstellen eines Mehrkomponenten-Vliesstoffs gemäß Anspruch 12, welches das Bilden von mehreren Öffnungen umfasst, die sich von dem Netzwerk von Flüssigkeit aufnehmenden Kanälen durch die Flüssigkeitsannahme- und die Flüssigkeitsverteilungsschicht erstrecken.

## Revendications

1. Tissu non tissé à composants multiples destiné à être utilisé pour des articles absorbants jetables, ledit tissu comprenant :
une couche de réception de liquide supérieure possédant une surface supérieure destinée à recevoir les liquides introduits dans ledit article absorbant, ladite couche supérieure comprenant des fibres choisies parmi des polymères thermoplastiques, des fibres thermoplastiques séparables, ou des fibres cellulosiques,
ladite surface supérieure de la couche supérieure définissant un ensemble de saillies verticales s'étendant au dessus d'un réseau de canaux de réception de liquide entourant lesdites saillies verticales ; et
une couche suivante de répartition de liquide juxtaposée à ladite couche supérieure, et hydroenchevêtrée avec cette dernière en une relation de transfert de liquide afin de recevoir le liquide provenant de ladite couche supérieure pour le distribuer vers ledit article absorbant,
ladite couche suivante comprenant un mélange de fibres comprenant : (1) des fibres choisies parmi le groupe constitué des polymères thermoplastiques, (2) des fibres thermoplastiques profilées, ou (3) des fibres cellulosiques.

2. Tissu non tissé à composants multiples selon la revendication 1, comprenant : une couche inférieure de rétention de liquide juxtaposée à ladite couche de répartition de liquide sur le côté de cette dernière opposé à ladite couche de réception de liquide, ladite couche de rétention de liquide étant hydroenchevêtrée en une relation de transfert de liquide avec ladite couche de répartition de liquide, ladite couche de rétention de liquide comprenant au moins en partie des fibres cellulosiques choisies parmi le groupe constitué de fibres de pâte de bois, de fibres de rayonne, ou de mélanges de ces dernières.

3. Tissu non tissé à composants multiples selon la revendication 2, dans lequel : ladite couche de rétention de liquide comprend un mélange desdites fibres cellulosiques et fibres de polymère thermoplastique possédant un denier compris entre environ 6 et 18.

4. Tissu non tissé à composants multiples selon la revendication 3, dans lequel : les polymères thermoplastiques sont choisis parmi le groupe constitué des polyoléfines, des polyesters, des polyamides, et de mélanges de ces derniers.

5. Tissu non tissé à composants multiples selon la revendication 4, dans lequel : les polyoléfines sont choisies parmi le groupe constitué du polypropylène, du polyéthylène, et de mélanges de ces derniers.

6. Tissu non tissé à composants multiples selon la revendication 4, dans lequel : les polyesters sont choisis parmi le groupe constitué du polyéthylène téréphtalate, du polybutylène téréphtalate, et de mélanges de ces derniers.

7. Tissu non tissé à composants multiples selon la revendication 4, dans lequel : le polyamide comprend le nylon.

8. Tissu non tissé à composants multiples selon la revendication 2, dans lequel : ladite couche suivante comprend un mélange de fibres comprenant : (1) des fibres possédant un denier d'environ 6 à 18 choisies parmi le groupe constitué des polymères thermoplastiques, (2) des fibres thermoplastiques profilées, ou (3) des fibres cellulosiques.

9. Tissu non tissé à composants multiples selon la revendication 1, dans lequel : ladite couche de répartition de liquide s'étend dans ledit ensemble de saillies définies par la surface supérieure de ladite couche de réception de liquide.

10. Tissu non tissé à composants multiples selon la revendication 2, dans lequel : une surface inférieure de ladite couche de rétention de liquide, opposée à ladite couche de répartition de liquide, est grattée en surface pour améliorer le gonflant.

11. Tissu non tissé à composants multiples selon la revendication 1, dans lequel : ledit tissu définit une pluralité d'ouvertures s'étendant depuis ledit réseau de canaux de réception du liquide au travers de ladite couche inférieure de répartition du liquide.

12. Procédé de fabrication d'un tissu non tissé à plusieurs composants, comprenant les étapes consistant à :
◆ former une bande fibreuse précurseur stratifiée comprenant une première couche fibreuse comprenant des fibres choisies parmi le groupe constitué du polypropylène, du polyester, du polyéthylène téréphtalate, et du nylon et possédant une masse surfacique comprise entre environ 0,010 et 0,030 kg/m² (0,5 et 1,5 oz/ yd²), et une deuxième couche fibreuse comprenant un mélange de fibres comprenant (1) des fibres ayant un denier allant d'environ 6 à 18 choisies parmi le groupe constitué des polyoléfines, des polyesters, et des polyamides, et (2) une fibre thermofusible, ladite deuxième couche possédant une masse surfacique comprise entre environ 0,010 et 0,020 kg/m² (0,5 et 1,0 oz/yd²) ;
◆ fournir un dispositif de transfert d'image tridimensionnelle possédant une surface de formation foraminée définissant un ensemble de dépressions superficielles ;
◆ positionner ladite bande fibreuse précurseur sur ledit dispositif de transfert d'image avec ladite première couche fibreuse positionnée de manière adjacente à ladite surface de formation foraminée, et
◆ hydroenchevêtrer ladite bande précurseur moyennant quoi ladite bande précurseur est imagée et formée sur ledit dispositif de transfert d'image, afin de former un tissu non tissé à composants multiples, ladite première couche fibreuse formant une couche de réception de liquide dudit tissu possédant un ensemble de saillies verticales s'étendant au dessus d'un réseau de canaux de réception de liquide, ledit ensemble de saillies correspondant audit ensemble de dépressions superficielles définies par ladite surface de formation foraminée, ladite deuxième couche fibreuse formant une couche de répartition de liquide dudit tissu juxtaposée à ladite couche de réception du liquide et hydroenchevêtrée à cette dernière en une relation de transfert de liquide.

13. Procédé de fabrication d'un tissu non tissé à composants multiples selon la revendication 12, comprenant :
la formation de ladite bande précurseur avec une troisième couche fibreuse comprenant au moins partiellement des fibres cellulosiques choisies parmi le groupe constitué de fibres de pâte de bois, de fibres de rayonne, et de mélanges de ces dernières, ladite deuxième couche fibreuse étant positionnée entre lesdites première et troisième couches fibreuses,
ladite étape d'hydroenchevêtrement comprenant la formation de ladite troisième couche fibreuse en tant que couche de rétention de liquide dudit tissu.

14. Procédé de fabrication d'un tissu non tissé à composants multiples selon la revendication 13, comprenant : la formation de ladite troisième couche fibreuse à partir d'un mélange desdites fibres cellulosiques et des fibres possédant un denier allant de 6 à 18 choisie parmi le groupe constitué des fibres polyesters, des fibres polyoléfines, et des fibres polyamides.

15. Procédé de fabrication d'un tissu non tissé à composants multiples selon la revendication 13, comprenant : la formation de ladite couche de réception du liquide contenant un polymère superabsorbant.

16. Procédé de fabrication d'un tissu non tissé à composants multiples selon la revendication 15, comprenant les étapes consistant à :
◆ disposer ledit polymère superabsorbant sous une forme fibreuse dans ladite troisième couche fibreuse de ladite bande précurseur, et
◆ sécher ledit tissu non tissé à température élevée afin d'activer ledit polymère superabsorbant.

17. Procédé de fabrication d'un tissu non tissé à composants multiples selon la revendication 13, comprenant l'étape consistant à : gratter la surface de ladite couche de rétention de liquide.

18. Procédé de fabrication d'un tissu non tissé à composants multiples selon la revendication 12, comprenant l'étape consistant à : former une pluralité d'ouvertures s'étendant depuis ledit réseau de canaux de réception de liquide au travers desdites couches de réception de liquide et de répartition de liquide.
